# EUROPEAN PATENT SPECIFICATION

(11) **EP 2 478 925 B1**
(45) Date of publication and mention of the grant of the patent: **16.04.2014**
(21) Application number: 11151565.6
(22) Date of filing: 20.01.2011
(51) Int. Cl.: A61M 16/06

(54) **Improved membrane cushion for facial mask for treating sleep disorders**
Verbessertes Membrankissen für Gesichtsmaske zur Behandlung von Schlafstörungen
Coussin de membrane amélioré pour masque facial afin de traiter les troubles du sommeil

(43) Date of publication of application: 25.07.2012
(73) Proprietor: Air Liquide Medical Systems, 92160 Antony (FR)
(72) Inventor: Alberici, Luca, 25128, Brescia (IT); Rivetti, Alberto, Rodengo Saiano (BS) 25050, Rovato (BS) (IT); Sandoni, Giuseppe, 25124, Brescia (IT); Lopez, Guillaume, 22560, Pleumeur Bodou (FR); Masserdotti, Fulvio, 25075, Brescia (IT)
(74) Representative: Pittis, Olivier

(56) References cited:
- EP-A1- 1 258 266
- EP-A2- 1 982 740
- US-A1- 2008 110 464

## Description

The invention concerns a cushion for a respiratory mask, in particular a facial mask, and a respiratory mask equipped with such a cushion for use in the treatment of respiratory conditions or diseases, such as obstructive sleep apnea, said cushion being made of soft material and comprising two superimposed flexible membranes ensuring efficient air tightness.

Masks, such as facial or nasal masks, are commonly used for delivering non-invasive positive pressure ventilation (NPPV) or for nasal continuous positive airway pressure (N-CPAP) therapy in sleep disordered breathing (SDB) conditions, such as obstructive sleep apnea (OSA). They typically deliver a flow of breathable gas for, or to assist in, patient respiration, especially during the night, i.e., when the patient is sleeping. Such a mask assembly typically comprises a rigid or semi-rigid hollow shell, also called a mask body, defining a breathing chamber that receives at least a part of the patient's nose and/or mouth, and further comprising a soft face-contacting cushion that comes into contact with the patient's face, and an optionally pivotable forehead support and a headgear for correctly positioning, maintaining and/or securing the mask on the head of a patient.

The shell of the mask or mask body is connected to a gas supply line which delivers a respiratory gas, such as air under pressure, into the breathing chamber of the shell through a gas opening or inlet orifice arranged in the wall of the mask body. The connection between the gas line and the mask body is usually obtained by means of a hollow connector comprising an internal passage for the gas. The connector can have an elbow-shape, i.e. be curved, or any other shape, and can also be rotatable around the axis of the inlet orifice. The soft face-contacting cushion often comprises two superimposed membranes, e.g. an inner membrane recovered by an outer membrane that ensures the air tightness all around the periphery of the cushion in contact with the user's face. These types of respiratory masks are known from documents such asEP-A-1737524, EP-A-1841481, EP-A-956069 or EP-A-1118346.

More precisely, when the mask is carried by the patient, the outer membrane comes into contact with the different regions of the user's face, i.e. the nasal bridge region, the chin region and the two lateral cheek regions that join the nasal bridge region to the chin region. As both membranes are made of a resilient flexible or soft material, the membranes are deformed under the traction forces exerted by the headgear of the mask, so as to match the profile and forms of the user's face, thereby ensuring the air tightness of the mask.

However, the existing masks equipped with such two-membrane cushions are not totally satisfying.

A problem that often exists is that leaks appear in the chin region of the user, i.e., the region of the user's face located below the mouth, during the night while the patient is sleeping, thus allowing for undesirable and unpleasant escape of the gas under pressure.

Those loses of gas under pressure that are due to the leaks, negatively impact the efficiency of the overall treatment and further cause some comfort issues for the patient.

Even though the problem is well known, controlling these air leaks is not an easy task as they depend on the patient's face shape, i.e. the patient's physiognomy, especially the width of the patient's nose. In other words, the leaks will vary from one patient to another.

Hence, the problem to be solved is to provide an improved cushion for respiratory masks, especially for facial masks, equipped in a manner to limit the gas leaks in the chin region when a patient is using the mask while sleeping, regardless of the patient's chin region profile or physiognomy.

In other words, the goal is to propose an improved cushion for respiratory masks that is able to ensure an efficient gas tightness whatever the patient's physiognomy, i.e. a cushion that can match various face shapes, especially an improved tightness in the chin region, and further a facial mask equipped with such an improved cushion.

The solution of the present invention concerns a cushion for respiratory mask, such as facial masks, having a three-dimensional shape with a central passage for receiving at least a part of the nose and mouth of a user, said cushion comprising at least a nasal bridge region, two cheek regions and a chin region, which come into contact respectively with the nasal bridge region, the cheek regions and the chin region of the user when the cushion is positioned on the user's face, said cushion comprising an inner membrane and an outer membrane, said inner and outer membranes being superimposed so that the outer membrane recovers the inner membrane and is in direct contact with the face of the user, said inner and outer membranes being made of a flexible material, the chin region of the inner membrane of the cushion comprises at least a central portion situated between two lateral portions, the central portion having a first thickness that is less than the second thickness of said lateral portions.

The cushion for respiratory masks can further comprise one or more of the following additional features:
- the inner and outer membranes are arranged on a cushion frame.
- the first thickness of the central portion of the inner membrane is less than 0,6 mm, preferably the first thickness is of at least 0,2 mm.
- the second thickness of the lateral portions of the inner membrane is more than 0,6 mm, preferably at least 0,7 mm, more preferably the second thickness is between 0,7 and 3 mm, advantageously less than 2 mm.
- the first thickness of the central portion of the inner membrane is of about 0,4 mm and the second thickness of the lateral portions of the inner membrane is of about 0,8 to 1 mm.
- intermediate portions are comprised between the central portion and each of the two lateral portions of the inner membrane.
- each intermediate portion has a progressively decreasing thickness from the second thickness to the first thickness.
- the central portion comprises several cuts.
- the cushion frame has a triangular shape or a saddle shape.
- the cushion frame comprises fixing means for fixing the cushion frame to a respiratory mask. Actually, the cushion frame can be adapted to be removably or permanently connected, via mechanical and/or adhesive fastening, to the mask body. Preferably, the cushion frame includes a base wall structured to be connected to the peripheral wall of mask body, for instance the wall of the cushion frame at its free end can have particular structure, such as a U-, Y-, V-shape or similar, that matches the peripheral border structure of the mask body so as to form together a tight connection. Such structures are well known in the art as taught, for instance, by documents EP-A-1118346, WO-A-9804310, WO-A-2006074513 or EP-A-1841481.
- the inner and outer membranes and the cushion frame are molded in one piece.
- the inner and outer membranes and the cushion frame are made of a resilient, elastomeric material, preferably of silicone.
- the outer membranes comprises a free curved edge projecting toward the interior of the cushion.

The invention also concerns a respiratory mask comprising a mask body with an internal chamber and a gas inlet orifice in fluid communication with said internal chamber, the internal chamber comprising a peripheral border, and the mask body further comprising a cushion according to the present invention that is fixed to the peripheral border of the mask body.

The mask according to the present invention can further comprise one or more of the following additional features:
- the mask is a facial mask.
- the mask further comprises a forehead support, a headgear and/or fixing means for fixing the headgear to the mask body, such as hooks, slots, buckles or similar.
- the forehead support is arranged on a holding arm connected to an expansion part of the mask body, and a rotating knob allows for modification of the angular position of the holding arm when said rotating knob is operated/rotated by a user.
- the expansion part is projecting upwardly and is further integral with the mask body, preferably the expansion part and the mask body are molded in one piece.
- the forehead support comprises one or several pillows of soft material with the pillows coming into contact with the forehead of the user.
- a hollow connector having an internal gas passage, such as an elbow-shape connector, is connected to the gas inlet orifice so as to be in fluid communication with the internal chamber of the mask.
- the hollow connector comprises an anti-asphyxia valve.

In choosing different thicknesses for the different portions of the chin region of the inner membrane of the cushion, it is possible to have a better control of the way the mask cushion fits with the chin region of the patient. Indeed, in doing so, the outer membrane can better match the chin region profile thus limiting and minimizing the gas leaks.

An embodiment of a cushion for a facial mask according to the present invention is shown in the enclosed Figures, among which :
- Figure 1 represents a facial mask according to the present invention when positioned on the patient's face,
- Figure 2 shows a cushion according to the present invention,
- Figure 3 is en enlarged view of the chin region of a first embodiment of a cushion according to the present invention,
- Figures 4 and 5 represent a second embodiment of the cushion of the present invention, and
- Figure 6 represents a third embodiment of the cushion of the present invention.

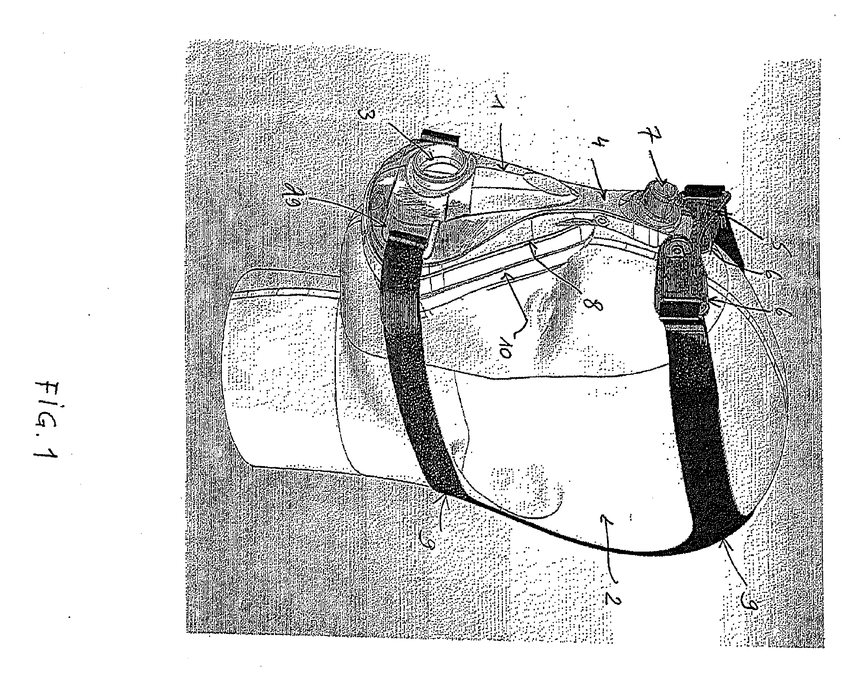

As illustrated in Figure 1, a respiratory facial mask according to the present invention comprises a rigid or semi-rigid hollow shell or mask body 1 defining an internal breathing chamber or volume, wherein respiratory gas, such as air under pressure, is introduced via an inlet port 3 to which is connected a gas feeding line, such a flexible gas conduit, by means of a tubular hollow connector (not shown). Preferably, the connector has a general elbow-shape and comprises an internal passage for the gas. The gas inlet orifice 3 is arranged at the center of the mask body 1 and through the mask body wall thereby allowing air under pressure to be introduced in the breathing chamber that receives the nose and mouth of the patient 2.

The mask body 1 is preferably made of a polymer material, such as polycarbonate (PC), polypropylene (PP), ABS, nylon or polystyrene (PS), and is configured so as to be able to receive at least a part of the patient's nose. In other words, the patient introduces his/her nose and mouth into the internal volume of the breathing chamber of the mask body 1 and breathes the pressurized gas contained therein. The mask body 1 has preferably a general triangular or quasi-triangular three-dimensional shape as visible in Figure 1, for example.

The mask body 1 further comprises an expansion part 4 arranged on the top of the mask body 1 and projecting upwardly from said mask body 1, i.e. projecting away from the external surface of the mask body 1. The expansion part 4 as well as the mask body 1 are made of polymeric material as described hereinbefore that is molded in one piece so as to obtain an expansion part 4 that is integral with the rest of the mask body 1.

Further, as represented in Figure 1, a forehead support 5 is connected to the mask body 1 by means of a preferably pivotable holding arm fixed to or carried by either the mask body 1 or the expansion part 4, preferably by the expansion part 4. The forehead support 5 can comprise one or several pillows 6 of soft and comfortable material that come into contact with the patient's forehead.

An acting piece (not visible in Fig. 1) is arranged in a traversing orifice of the expansion part 4 and is mobile, preferably in translation, in said traversing orifice so as to cooperate with the holding arm for pivoting said holding arm, when said acting piece moves in the traversing orifice of the expansion part 4. Further, the forehead support 5 can also pivot with respect to the holding arm 4.

Actually, the backward or forward motion of the acting piece in the traversing orifice is obtained by manual rotation by the user of a rotating knob 7 cooperating with the acting piece, when said rotating knob 7 is manually operated, i.e. turned clockwise or counterclockwise, by the user. The maximum course of the knob 6 is about 360° or less.

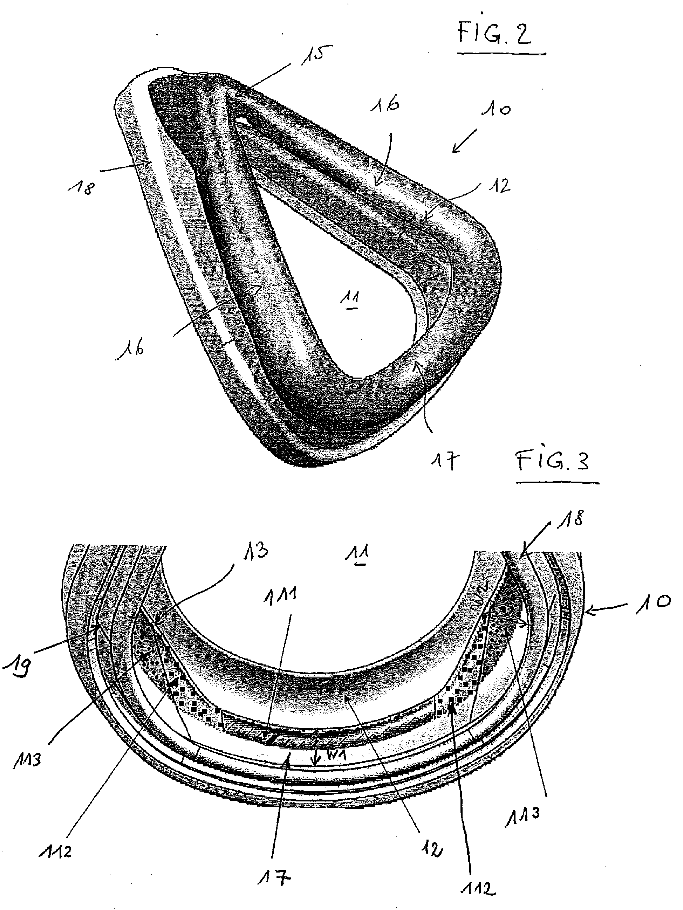

Furthermore, in order to ensure a tight positioning of the nasal mask on the patient's face and to increase the comfort for the patient 2, the peripheral border or edge 8 of the mask body 1 comprises a cushion 10 made of soft, resilient, elastomeric material that comes into contact with the patient's face as detailed in the various embodiments of Figures 2-6

As represented in Figure 2, said cushion 10 has a central aperture 11 for receiving at least a part of the patient's nose and mouth. The central aperture 11 of the cushion 10 is located vis a vis with the internal chamber of the mask body 1, i.e. they are facing each other. The cushion 10 arranged on the peripheral border 8 of the internal chamber of the mask body 1 comprises two superimposed flexible membranes 12, 13, for example made of silicone, ensuring an air tightness so as to minimize air leaks.

Such kinds of cushion 10 and mask body 1 structures are well-known in the art and taught by many documents, such as EP-A-1334742, EP-A-264772, EP-A-956069, EP-A-874667, US-A-2,931,356 , EP-A-1479406 or EP1982740A2.

More precisely, the border 8 and the cushion 10 have a general triangular or saddle-shape structure so as to match the contours of the nasal region, including the upper nasal bridge region, the cheek regions on both sides of the nose, and the lower chin region of the patient 2 situated under the patient's mouth.

Further, a headgear 9 comprising straps can be connected to the mask body 1, by fixing means 29, such as hooks, buckles or slots, for fixing the straps of the headgear 9. Thereby, the mask is maintained in a desired position on the face of the patient during use of the mask and thus an efficient treatment of sleep disorders, such as OSA or similar disorders, is obtained.

The headgear-fixing means 19 can be made integral with the mask body 1. Such structures are well known in the art and disclosed in many documents such as, for example, EP-A-1334742, EP-A-462701, EP-A-1985327 or EP-A-956069.

The shell or mask body 1 is fluidly linked to a gas supply line, such as a flexible hose or conduit conveying respiratory gas to the mask, by means of a tubular hollow connector, which delivers a respiratory gas, such as air under pressure, into the breathing chamber of the shell 1 through the gas inlet 3 arranged in the mask body 1. Gas under pressure, such as air, can be produced and delivered in a BPAP or CPAP-type apparatus.

Furthermore, the inner and outer membranes 12, 13 are made of flexible material, such as a resilient, elastomeric material like silicone, so that they can fit with the patient's face contours. The frame 18 bearing said inner and outer membranes 12, 13 is preferably also made of the same resilient, elastomeric material like silicone.

According to the present invention, the chin region 17 of the inner membrane 13 of the cushion 10 is divided in 3 sub-regions comprising a central portion 111 and two opposite lateral portions 113, which are separated one from the other by two intermediate portions 112, as detailed in Figure 3. In other words, each intermediate portion 112 is comprised "in sandwich" between the central portion 111 and one of the lateral portions 113.

Further, the central portion 111 has a first thickness, for example of about 0,5 mm, that is less than the second thickness, for example 0,8 mm, of each lateral portions 113. Indeed, using different thicknesses in the chin region 17 of the inner membrane 13 as shown in Figure 3, allows for obtaining a more efficient gas tightness in the chin region as the inner membrane 13 is able to better cooperate with the outer membrane 12 which fits or matches the contours of the patient's chin region 17 when the mask equipped with such a cushion 10 is applied on the patient's face.

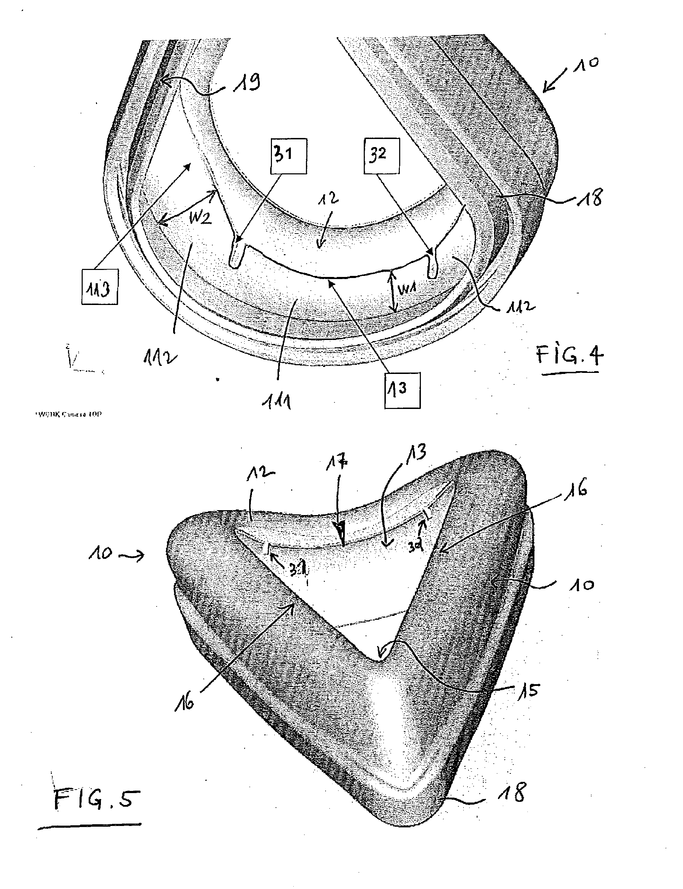

In a second embodiment shown in Figure 4 and 5, the central portion 111 having the first thickness of the inner membrane 13 comprises 2 cuts 31, 32 situated at the junction of said central portion 111 and each of the intermediate portions 112. This allows obtaining a more flexible and more easily deformable and bendable central portion 111 when the mask is positioned on the patient's face.

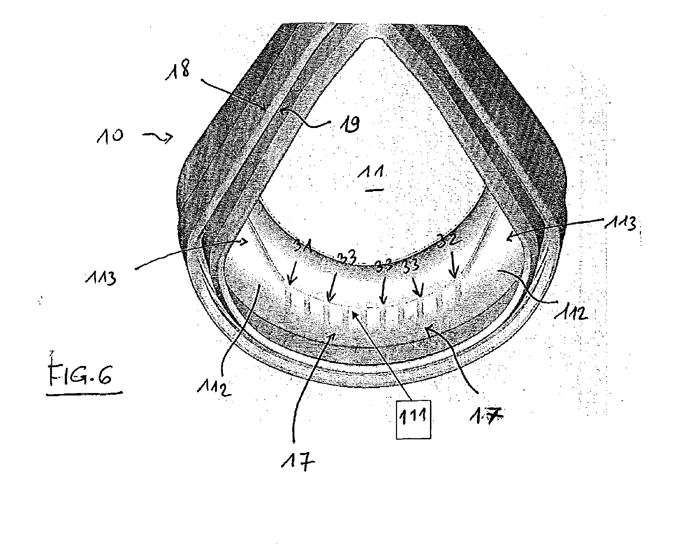

In a third embodiment, the central portion 111 having the first thickness comprises the 2 cuts 31, 32 made in the inner membrane 13 and situated at the junction of said central portion 111 and each of the intermediate portions 112 as in the second embodiment, but also one or several additional cuts 33 made in said central portion 111 between the cuts 31, 32 as shown in Figure 6. This allows increasing more the flexible of the inner membrane 13 and hence the comfort for the user.

In any cases, the cushion frame 10 also comprises fixing means 19, such as a particular configuration (see Fig. 4 or 6 for instance) of its peripheral border, for fixing the cushion frame 10 to a respiratory mask body 1.Actually, the cushion frame can be adapted to be removably or permanently connected, via mechanical and/or adhesive fastening, to the mask body. Preferably, the cushion frame includes a base wall structured to be connected to the peripheral wall of mask body, for instance the wall of the cushion frame at its free end can comprise a particular structure or configuration, such as a U-, Y-, V-shape or similar, that matches and/or cooperate with the peripheral border structure of the mask body so as to form together a tight connection. Such structures are well known in the art as taught, for instance, by documents EP-A-1118346, WO-A-9804310, WO-A-2006074513 or EP-A-1841481.

Further, as represented in Figure 3 and 4, the width W1 of the inner membrane 13 in the central portion 111 in the chin region 17 is less than the width W2 of said inner membrane 13 in the lateral 113 and/or intermediate portions 112. For example, the width W1 in the central portion 111 can be of about 5 mm, whereas the width W2 in the lateral 113 and/or intermediate portions 112 is of at least 10 mm.

Furthermore, the inner and outer membranes 12, 13 and the cushion frame 18 are preferably molded in one-piece and/or made of a resilient, elastomeric material such as silicone.

In addition, the outer membrane 12 advantageously comprises a free curved edge 21 projecting toward the interior of the cushion. Therefore, the width of the outer membrane 12 should be larger than the one of the inner membrane 13 so that said outer membrane 12 can totally cover the inner membrane as shown in Figures 3 to 5.

While the facial mask of the present invention can be used in any manner in which facial masks are necessary, the facial mask of the present invention is particularly useful in a method for treatment of a respiratory disorder or condition, for example, in non-invasive positive pressure ventilation (NPPV) or in a continuous positive airway pressure (CPAP) therapy of sleep disordered breathing (SDB) conditions, such as, for example, obstructive sleep apnea (OSA).

## Claims

1. Cushion (10) for respiratory mask, having a three-dimensional shape with a central passage (11) for receiving at least a part of the nose and mouth of a user, said cushion (10) comprising at least a nasal bridge region (15), a chin region (17) and two cheek regions (16), which come into contact with the nasal bridge region, the cheek regions and the chin region of the user, respectively, when the cushion (10) is positioned on the user's face, said cushion (10) further comprising an inner membrane (13) and an outer membrane (12), said inner and outer membranes (12, 13) being superimposed so that the outer membrane (12) covers the inner membrane (13) and is in direct contact with the face of the user (2), said inner and outer membranes (12, 13) being made of a flexible material, the chin region (17) of the inner membrane (13) of the cushion comprising at least a central portion (111) situated between two lateral portions (113), the central portion (111) having a first thickness that is less than the second thickness of said lateral portions (113), wherein :
- the first thickness of the central portion (111) of the inner membrane (13) is of less than 0,6 mm,
- intermediate portions (112) are comprised between the central portion (111) and each of the two lateral portions (113) of the inner membrane, said intermediate portions (112) having a progressively decreasing thickness from the second thickness to the first thickness, **characterized in that**,
- the second thickness of the lateral portions (113) of the inner membrane (13) is greater than 0,7 mm and less or equal to 1 mm, and
- the width (w1) of the inner membrane (13) in the central portion (111) in the chin region (17) is less than the width (w2) of said inner membrane (13) in the lateral (113) and/or intermediate portions (112).

2. Cushion according to Claim 1, **characterized in that** the inner and outer membranes (12, 13) are arranged on a cushion frame (18).

3. Cushion according to any one of the preceding Claims, **characterized in that** the first thickness of the central portion (111) of the inner membrane (13) is of at least 0,2 mm.

4. Cushion according to any one of the preceding Claims, **characterized in that** the first thickness of the central portion (111) of the inner membrane (13) is of about 0,4 mm and the second thickness of the lateral portions (113) of the inner membrane (13) is of about 0,8 to 1 mm.

5. Cushion according to any one of the preceding Claims, **characterized in that** the central portion (111) comprises several cuts (31, 32, 33).

6. Cushion according to any one of the preceding Claims, **characterized in that** the cushion frame (10) has a triangular shape or a saddle shape and/or the cushion frame (10) comprises fixing means (19) for fixing the cushion frame (10) to a respiratory mask body (1).

7. Cushion according to any one of the preceding Claims, **characterized in that** the inner and outer membranes (12, 13) and the cushion frame (18) are molded in one piece.

8. Cushion according to any one of the preceding Claims, **characterized in that** the inner and outer membranes (12, 13) and the cushion frame (18) are made of silicone.

9. Cushion according to any one of the preceding Claims, **characterized in that** the outer membranes (12, 13) comprises a free curved edge (21) projecting toward the interior of the cushion (10).

10. Respiratory mask comprising a mask body (1) with an internal chamber and a gas inlet orifice (3) in fluid communication with said internal chamber, the internal chamber comprising a peripheral border (8), and the mask body (1) further comprising a cushion (10) according to any one of the preceding Claims that is fixed to the peripheral border (8) of the mask body (1).

11. Respiratory mask according to claim 10, **characterized in that** the mask is a facial mask.

12. Respiratory mask according to any one of Claims 10 or 11, **characterized in that** it further comprises a forehead support (5), a headgear (9) and/or connecting means (29) for fixing the headgear (9) to the mask body (1).

13. Respiratory mask according to any one of Claims 10 to 12, **characterized in that** the forehead support is arranged on a holding arm connected to an expansion part of the mask body, and a rotating knob allows for modification of the angular position of the holding arm when said rotating knob is operated/rotated by a user.

14. Respiratory mask according to any one of Claims 10 to 13, **characterized in that** a hollow connector having an internal gas passage is connected to the gas inlet orifice so as to be in fluid communication with the internal chamber of the mask, said hollow connector comprising an anti-asphyxia valve.

## Patentansprüche

1. Polster (10) für eine Atmungsmaske, das eine dreidimensionale Form mit einem mittleren Durchlass (11) zur Aufnahme mindestens eines Teils der Nase und des Mundes eines Benutzers aufweist, wobei das Polster (10) mindestens einen Nasenwurzelbereich (15), einen Kinnbereich (17) und zwei Wangenbereiche (16) umfasst, die mit dem Nasenwurzelbereich, den Wangenbereichen beziehungsweise dem Kinnbereich des Benutzers in Kontakt kommen, wenn das Polster (10) auf dem Gesicht des Benutzers positioniert ist, wobei das Polster (10) ferner eine innere Membran (13) und eine äußere Membran (12) umfasst, wobei die innere und die äußere Membran (12, 13) derart übereinanderliegen, dass die äußere Membran (12) die innere Membran (13) abdeckt und in direktem Kontakt mit dem Gesicht des Benutzers (2) steht, wobei die innere und die äußere Membran (12, 13) aus einem flexiblen Material hergestellt sind, wobei der Kinnbereich (17) der inneren Membran (13) des Polsters mindestens einen mittleren Abschnitt (111) umfasst, der sich zwischen zwei Seitenabschnitten (113) befindet, wobei der mittlere Abschnitt (111) eine erste Dicke aufweist, die geringer als die zweite Dicke der Seitenabschnitte (113) ist, wobei:
die erste Dicke des mittleren Abschnitts (111) der inneren Membran (13) weniger als 0,6 mm beträgt,
zwischen dem mittleren Abschnitt (111) und jedem der zwei Seitenabschnitte (113) der inneren Membran Zwischenabschnitte (112) enthalten sind, wobei die Zwischenabschnitte (112) von der zweiten Dicke zur ersten Dicke eine progressiv abnehmende Dicke aufweisen,
**dadurch gekennzeichnet, dass**
die zweite Dicke der Seitenabschnitte (113) der inneren Membran (13) größer als 0,7 mm und kleiner gleich 1 mm ist und
die Breite (w1) der inneren Membran (13) im mittleren Abschnitt (111) im Kinnbereich (17) kleiner als die Breite (w2) der inneren Membran (13) in den Seiten- (113) und/oder in den Zwischenabschnitten (112) ist.

2. Polster nach Anspruch 1, **dadurch gekennzeichnet, dass** die innere und die äußere Membran (12, 13) auf einem Polsterrahmen (18) angeordnet sind.

3. Polster nach einem der vorhergehenden Ansprüche, **dadurch gekennzeichnet, dass** die erste Dicke des mittleren Abschnitts (111) der inneren Membran (13) mindestens 0,2 mm beträgt.

4. Polster nach einem der vorhergehenden Ansprüche, **dadurch gekennzeichnet, dass** die erste Dicke des mittleren Abschnitts (111) der inneren Membran (13) mindestens 0,4 mm beträgt und die zweite Dicke der Seitenabschnitte (113) der inneren Membran (13) etwa 0,8 bis 1 mm beträgt.

5. Polster nach einem der vorhergehenden Ansprüche, **dadurch gekennzeichnet, dass** der mittlere Abschnitt (111) mehrere Einschnitte (31, 32, 33) umfasst.

6. Polster nach einem der vorhergehenden Ansprüche, **dadurch gekennzeichnet, dass** der Polsterrahmen (10) eine dreieckige Form oder eine Sattelform aufweist und/oder der Polsterrahmen (10) Befestigungsmittel (19) zum Befestigen des Polsterrahmens (18) an einem Atemmaskenkörper (1) umfasst.

7. Polster nach einem der vorhergehenden Ansprüche, **dadurch gekennzeichnet, dass** die innere und die äußere Membran (12, 13) und der Polsterrahmen (18) einstückig geformt sind.

8. Polster nach einem der vorhergehenden Ansprüche, **dadurch gekennzeichnet, dass** die innere und die äußere Membran (12, 13) und der Polsterrahmen (18) aus Silikon hergestellt sind.

9. Polster nach einem der vorhergehenden Ansprüche, **dadurch gekennzeichnet, dass** die äußere Membran (12) einen freien gewölbten Rand (21) umfasst, der zum Inneren des Polsters (10) ragt.

10. Atemmaske, einen Maskenkörper (1) mit einer Innenkammer und einer Gaseinlassöffnung (3) in Fluidverbindung mit der Innenkammer umfassend, wobei die Innenkammer eine umlaufende Einfassung (8) aufweist und der Maskenköper (1) ferner ein Polster (10) nach einem der vorhergehenden Ansprüche umfasst, das an der umlaufenden Einfassung (8) des Maskenköpers (1) befestigt ist.

11. Atemmaske nach Anspruch 10, **dadurch gekennzeichnet, dass** die Maske eine Gesichtsmaske ist.

12. Atemmaske nach einem der Ansprüche 10 oder 11, **dadurch gekennzeichnet, dass** sie ferner eine Stirnauflage (5), ein Kopfgeschirr (9) und/oder Anschlussmittel (29) zum Befestigen des Kopfgeschirrs (9) am Maskenkörper (1) umfasst.

13. Atemmaske nach einem der Ansprüche 10 bis 12, **dadurch gekennzeichnet, dass** die Stirnauflage an einem Haltearm angeordnet ist, der an einem Ausdehnungsteil des Maskenköpers angeschlossen ist und ein Drehknopf die Veränderung der Winkelposition des Haltearms gestattet, wenn der Drehknopf durch einen Benutzer bedient/gedreht wird.

14. Atemmaske nach einem der Ansprüche 10 bis 13, **dadurch gekennzeichnet, dass** an die Gaseinlassöffnung ein Hohlanschluss mit einem inneren Gasdurchlass angeschlossen ist, so dass er mit der Innenkammer der Maske in Fluidverbindung steht, wobei der Hohlanschluss ein Anti-Asphyxieventil umfasst.

## Revendications

1. Coussin (10) pour masque respiratoire, ayant une forme tridimensionnelle avec un passage central (11) destiné à recevoir au moins une partie du nez et de la bouche d'un utilisateur, ledit coussin (10) comprenant au moins une région d'arête nasale (15), une région de menton (17) et deux régions de joues (16), qui viennent au contact de la région d'arête nasale, des régions de joues et de la région de menton de l'utilisateur, respectivement, lorsque le coussin (10) est positionné sur le visage de l'utilisateur, ledit coussin (10) comprenant en outre une membrane interne (13) et une membrane externe (12), lesdites membranes interne et externe (12, 13) étant superposées de sorte que la membrane externe (12) couvre la membrane interne (13) et soit en contact direct avec le visage de l'utilisateur (2), lesdites membranes interne et externe (12, 13) étant faites d'un matériau flexible, la région de menton (17) de la membrane interne (13) du coussin comprenant au moins une portion centrale (111) située entre deux portions latérales (113), la portion centrale (111) ayant une première épaisseur qui est inferieure à la deuxième épaisseur desdites portions latérales (113), dans lequel :
- la première épaisseur de la portion centrale (111) de la membrane interne (13) est inférieure à 0,6 mm,
- des portions intermédiaires (112) sont comprises entre la portion centrale (111) et chacune des deux portions latérales (113) de la membrane interne, lesdites portions intermédiaires (112) ayant une épaisseur qui diminue progressivement de la deuxième épaisseur à la première épaisseur, **caractérisé en ce que**,
- la deuxième épaisseur des portions latérales (113) de la membrane interne (13) est supérieure à 0,7 mm et inférieure ou égale à 1 mm, et
- la largeur (w1) de la membrane interne (13) dans la portion centrale (111) dans la région de menton (17) est inférieure à la largeur (w2) de ladite membrane interne (13) dans les portions latérales (113) et/ou intermédiaires (112).

2. Coussin selon la revendication 1, **caractérisé en ce que** les membranes interne et externe (12, 13) sont agencées sur un cadre de coussin (18).

3. Coussin selon l'une quelconque des revendications précédentes, **caractérisé en ce que** la première épaisseur de la portion centrale (111) de la membrane interne (13) est d'au moins 0,2 mm.

4. Coussin selon l'une quelconque des revendications précédentes, **caractérisé en ce que** la première épaisseur de la portion centrale (111) de la membrane interne (13) est d'environ 0,4 mm et la deuxième épaisseur des portions latérales (113) de la membrane interne (13) est d'environ 0,8 à 1 mm.

5. Coussin selon l'une quelconque des revendications précédentes, **caractérisé en ce que** la portion centrale (111) comprend plusieurs découpes (31, 32, 33).

6. Coussin selon l'une quelconque des revendications précédentes, **caractérisé en ce que** le cadre de coussin (10) présente une forme triangulaire ou une forme de selle et/ou le cadre de coussin (10) comprend un moyen de fixation (19) permettant de fixer le cadre de coussin (18) à un corps de masque respiratoire (1).

7. Coussin selon l'une quelconque des revendications précédentes, **caractérisé en ce que** les membranes interne et externe (12, 13) et le cadre de coussin (18) sont moulés d'une seule pièce.

8. Coussin selon l'une quelconque des revendications précédentes, **caractérisé en ce que** les membranes interne et externe (12, 13) et le cadre de coussin (18) sont faits de silicone.

9. Coussin selon l'une quelconque des revendications précédentes, **caractérisé en ce que** la membrane externe (12) comprend un bord incurvé libre (21) se projetant vers l'intérieur du coussin (10).

10. Masque respiratoire comprenant un corps de masque (1) avec une chambre interne et un orifice d'admission de gaz (3) en communication fluidique avec ladite chambre interne, la chambre interne comprenant un bord périphérique (8), et le corps de masque (1) comprenant en outre un coussin (10) selon l'une quelconque des revendications précédentes, qui est fixé au bord périphérique (8) du corps de masque (1).

11. Masque respiratoire selon la revendication 10, **caractérisé en ce que** le masque est un masque facial.

12. Masque respiratoire selon l'une quelconque des revendications 10 ou 11, **caractérisé en ce qu'**il comprend en outre un support frontal (5), un harnais (9) et/ou un moyen de raccordement (29) pour fixer le harnais (9) au corps de masque (1).

13. Masque respiratoire selon l'une quelconque des revendications 10 à 12, **caractérisé en ce que** le support frontal est agencé sur un bras de support raccordé à une partie d'expansion du corps de masque, et un bouton rotatif permet la modification de la position angulaire du bras de support lorsque ledit bouton rotatif est actionné/tourné par un utilisateur.

14. Masque respiratoire selon l'une quelconque des revendications 10 à 13, **caractérisé en ce qu'**un raccord creux ayant un passage de gaz interne est raccordé à l'orifice d'entrée de gaz de façon à être en communication fluidique avec la chambre interne du masque, ledit raccord creux comprenant une valve anti-asphyxie.
